# FASCICULE DE BREVET EUROPEEN

(11) **EP 1 112 753 B1**
(45) Date de publication et mention de la délivrance du brevet: **14.07.2004**
(21) Numéro de dépôt: 01400001.2
(22) Date de dépôt: 02.01.2001
(51) Int. Cl.: A61L 27/08, A61L 27/30

(54) **Implant d'interposition mobile entre deux surfaces osseuses**
Mobiles Implantat für den Einsatz zwischen zwei Knochenoberflächen
Mobile implant for interposition between two osseous surfaces

(30) Priorité: 30.12.1999 FR 9916791
(43) Date de publication de la demande: 04.07.2001
(73) Titulaire: Bioprofile, 38028 Grenoble Cedex 1 (FR)
(72) Inventeur: Hassler, Michel, 38330 Saint-Ismier (FR); Pequignot, Jean-Pierre, 06000 Nice (FR); Real, Cécile, 38000 Grenoble (FR); Huet, Olivier, 38000 Grenoble (FR)
(74) Mandataire: Micheli & Cie

(56) Documents cités:
- US-A- 3 623 164
- US-A- 3 707 006
- US-A- 5 458 647

## Description

La présente invention concerne, de manière générale, un implant destiné à être introduit dans un patient. Plus particulièrement, l'implant selon l'invention vise à restituer la fonctionnalité d'une articulation rendue défectueuse et douloureuse par l'arthrose, le rhumatisme ou un accident.

On connaît des implants, du type prothèses, pour remplacer des os ou parties d'os. La plupart d'entre eux sont composés d'une pièce mâle et d'une pièce femelle. L'une au moins des pièces mâle et femelle comporte une tige qui vient s'engager dans une partie osseuse adjacente à l'os ou partie d'os que l'on souhaite remplacer, afin de maintenir fermement la prothèse contre cette partie osseuse. A cet effet, un trou est pratiqué dans le fût de la partie osseuse en retirant notamment de la moelle osseuse. La tige est ensuite introduite dans ledit trou, et fixée aux parois de celui-ci au moyen d'un ciment, tel qu'une résine polymétacrylate. En variante, les dimensions du trou dans le fût de la partie osseuse sont adaptées pour permettre l'engagement à force de la tige dans le trou.

Des exemples de prothèse ayant une telle tige de fixation sont décrits dans les documents US-A-3 707 006 et US-A-5 458 647.

Ces prothèses présentent plusieurs inconvénients majeurs.

En premier lieu, les opérations de fixation de la prothèse sont très délicates à mettre en oeuvre. Elles nécessitent notamment d'ajuster très précisément en dimension et en orientation le trou dans lequel la tige de la prothèse vient s'engager. Une mauvaise orientation du trou peut entraîner une usure prématurée de la pièce mâle contre la pièce femelle, ainsi qu'une mauvaise cinématique d'articulation. En outre, ces opérations de fixation sont irréversibles et ne laissent pas la possibilité au chirurgien de remplacer la prothèse en cas d'usure, sauf à créer des dommages conséquents sur le ou les os auxquels elle est fixée.

Ces prothèses requièrent d'autre part que l'on enlève, pour leur implantation dans le patient, tout ou partie de la moelle osseuse de l'os auquel la prothèse est fixée, pour former le trou.

Un autre inconvénient réside dans le fait que de telles prothèses présentent un risque important de descellement, après leur implantation dans le patient. Le descellement d'une prothèse consiste dans le détachement de la prothèse de l'os auquel elle était fixée. La prothèse ainsi devenue libre perd sa stabilité, c'est-à-dire qu'elle peut venir se placer dans une position inappropriée et gênante pour le patient. En outre, elle vient frotter contre les os avoisinants, ce qui entraîne des phénomènes d'usure. En fonction du matériau constituant la prothèse, de tels frottements ont généralement pour effet d'endommager la prothèse ou les os avoisinants. En particulier, lorsque la prothèse est conçue en un matériau plus dur que l'os, elle peut perforer un os et migrer à l'intérieur de celui-ci. Lorsque, à l'inverse, la prothèse s'use au contact des os avoisinants, des débris de prothèse sont produits dans le patient et peuvent entraîner des douleurs aiguës, notamment dans le cas d'une prothèse métallique.

Le brevet américain US-A-4 166 292 décrit une prothèse de genou dans laquelle une partie fémorale et une partie tibiale s'articulent entre elles de manière à reproduire les mouvements naturels du genou. Les parties fémorale et tibiale sont respectivement fixées au fémur et au tibia du patient. Les surfaces de la prothèse en regard du fémur et du tibia sont pourvues, à cet effet, de moyens de fixation du type rainures et nervures.

Le brevet américain US-A-4 936 860 enseigne une prothèse de scaphoïde destinée à être positionnée entre le trapèze et le semi-lunaire, en remplacement du scaphoïde du patient. Cette prothèse comporte, sur sa surface externe, une saillie adaptée pour s'engager dans un évidement correspondant pratiqué dans le trapèze, et un trou apte à recevoir un fil de suture permettant de lier la prothèse à des ligaments avoisinants. De tels moyens de fixation visent à conférer une stabilité suffisante à la prothèse dans le poignet du patient, pour empêcher que la prothèse ne sorte de son logement.

Les implants décrits dans les deux documents précités nécessitent des opérations relativement délicates pour fixer l'implant à un os ou à des ligaments.

La présente invention vise à remédier à ces inconvénients.

A cette fin, il est prévu un implant destiné à servir de pièce d'interposition entre des surfaces osseuses d'un patient, caractérisé en ce qu'au moins sa surface externe comprend du pyrocarbone, et en ce qu'il est dépourvu de tout moyen de fixation, de telle sorte que l'implant reste libre par rapport auxdites surfaces osseuses lorsqu'il est introduit dans le patient.

Les présents inventeurs ont ainsi découvert qu'il est possible d'obtenir un implant qui reste stable dans le patient et qui n'engendre pas de phénomènes d'usure, sans qu'il soit nécessaire de prévoir des moyens de fixation pour maintenir l'implant contre un os ou contre un ligament. Une fois introduit dans le patient, l'implant reste sensiblement mobile dans son logement, entre les surfaces osseuses. Il en résulte pour le patient un plus grand confort, puisqu'en laissant l'implant libre par rapport aux surfaces osseuses on augmente les degrés de liberté autorisés pour les mouvements du patient. En outre, la mobilité de l'implant permet, en entretenant des mouvements de glissement de l'implant par rapport aux surfaces osseuses, d'éviter que les tissus ligamentaires environnants ne viennent se coller à l'implant dans un processus de cicatrisation naturel.

Si le logement naturel entre les surfaces osseuses n'est pas adapté à la forme et aux mouvements de l'implant, le logement de l'implant est préalablement préparé en rognant de manière appropriée les surfaces osseuses.

Etant donné que l'implant est susceptible de se mouvoir dans son logement, les frottements entre l'implant et les os avoisinants peuvent être importants lors de mouvements effectués par le patient. De manière générale, il était admis, avant la présente invention, que de tels frottements devaient être réduits le plus possible, afin d'éviter que l'implant ou les os avoisinants ne s'usent au contact les uns des autres. C'est d'ailleurs l'une des raisons pour lesquelles, outre le problème de stabilité, les prothèses de la technique antérieure sont liées à un os ou à des ligaments.

Pour résoudre le problème d'usure résultant de la mobilité de l'implant, la présente invention utilise le pyrocarbone comme matériau de fabrication de l'implant.

Le pyrocarbone est un matériau connu de l'homme du métier pour ses propriétés de biocompatibilité. Il est déjà employé pour fabriquer des prothèses destinées à être fixées à un os. Dans de telles prothèses, la surface en pyrocarbone en contact avec l'os est généralement poreuse, pour permettre à l'os de remplir les pores, et, par là même, renforcer sa liaison avec la prothèse, comme décrit par exemple dans le document US-A-3 707 006.

A la différence des prothèses connues à ce jour, l'implant selon la présente invention utilise le pyrocarbone dans une fonction de frottement contre les os avoisinants, et non pas pour former une surface de fixation. Les présents inventeurs ont découvert que le pyrocarbone présente un très bon coefficient de frottement avec l'os, si bien que ni l'implant ni les os avec lesquels il est en contact ne s'usent lors de mouvements effectués par le patient. L'implant peut ainsi rouler ou glisser sur les os avoisinants sans engendrer de dommages. D'autre part, l'utilisation du pyrocarbone contribue à améliorer la stabilité de l'implant, par le fait qu'elle autorise un contact peu contraignant entre l'implant et les os, de sorte qu'il existe peu de chances que l'implant puisse être éjecté de son logement lors d'un mouvement effectué par le patient.

Une raison principale au bon comportement du pyrocarbone dans sa fonction de frottement avec l'os réside dans le fait que ce matériau présente un module d'élasticité, dit également module d'Young, approximativement compris entre 10 et 35 GPa, c'est-à-dire sensiblement voisin de celui de l'os. Ainsi, lorsque l'implant est en appui sur les os avoisinants, et vice-versa, les charges réciproques reçues par l'implant et les os s'équilibrent. Ces derniers reçoivent par conséquent des contraintes du même niveau que celles pour lesquelles ils ont été conçus. Si l'implant était, au contraire, conçu en un matériau bien plus dur que l'os, il tendrait à user, voire perforer, les os avoisinants. Dans le cas d'un matériau trop mou, les os avoisinants se dénatureraient, perdraient de leur substance en se décalcifiant, et pourraient donc s'user très facilement.

Par conséquent, bien que le pyrocarbone constitue le matériau préféré pour fabriquer l'implant selon l'invention, d'autres matériaux biocompatibles pourraient être utilisés à la place du pyrocarbone, à la condition qu'ils aient un module d'élasticité approximativement compris entre 10 et 35 GPa.

Selon une autre caractéristique particulièrement avantageuse de l'invention, la surface externe de l'implant est polie, afin de réduire encore davantage le coefficient de frottement. La surface externe est de préférence conçue essentiellement en pyrocarbone. En variante, l'implant tout entier est conçu en pyrocarbone.

L'implant selon l'invention peut présenter une forme simple, puisque, d'une part, aucun moyen de fixation n'est prévu sur sa surface externe et, d'autre part, le logement dans lequel il est introduit est préalablement préparé pour avoir une forme adaptée à celle de l'implant.

L'implant selon la présente invention peut notamment servir d'articulation entre deux os, ou de prothèse destinée à remplacer un os ou une partie d'os.

Ainsi, par exemple, lorsqu'une articulation entre deux os est devenue douloureuse, les surfaces osseuses en regard, ou seulement l'une d'entre elles, sont rognées afin de ménager un logement pour l'implant. L'implant est ensuite placé dans son logement, pour servir de pièce d'interposition et d'articulation entre les deux os.

Dans le cas où l'implant selon l'invention est utilisé en tant que prothèse, l'os ou la partie d'os défectueux est retiré, puis un logement de forme adaptée à celle de l'implant est préparé en rognant un ou plusieurs os adjacents à l'os ou la partie d'os à remplacer. L'implant est alors introduit dans le logement, sans être fixé, pour restituer la fonctionnalité de l'os défectueux.

D'autres caractéristiques et avantages de la présente invention apparaîtront à la lecture de la description détaillée suivante de plusieurs modes de réalisation, faite en référence aux dessins annexés dans lesquels :
- les figures 1A à 1D représentent respectivement en perspective et en vue de section dans trois plans mutuellement perpendiculaires un implant selon un premier mode de réalisation de la présente invention ;
- les figures 2A à 2C représentent, en vue de section dans les trois plans mutuellement perpendiculaires précités, un implant selon un second mode de réalisation de la présente invention ;
- les figures 3A à 3C représentent, en vue de section dans les trois plans mutuellement perpendiculaires précités, un implant selon un troisième mode de réalisation de la présente invention ;
- les figures 4A à 4C représentent, en vue de section dans les trois plans mutuellement perpendiculaires précités, un implant selon un quatrième mode de réalisation de la présente invention ;
- les figures 5A à 5D représentent, en vue de section dans les trois plans mutuellement perpendiculaires précités, un implant selon un cinquième mode de réalisation de la présente invention ;
- les figures 6A à 6C représentent, en vue de section dans les trois plans mutuellement perpendiculaires précités, un implant selon un sixième mode de réalisation de la présente invention ;
- les figures 7A à 7C représentent, en vue de section dans les trois plans mutuellement perpendiculaires précités, un implant selon un septième mode de réalisation de la présente invention ;
- les figures 8A à 8C représentent, en vue de section dans les trois plans mutuellement perpendiculaires précités, un implant selon un huitième mode de réalisation de la présente invention ;
- la figure 9A est une vue de dessus schématique d'une main droite comportant des implants selon l'invention ;
- la figure 9B est une vue de côté en section plane d'un implant selon l'invention introduit dans le doigt d'un patient ;
- la figure 10A est une vue de dessus schématique d'un poignet gauche ;
- les figures 10B et 10C sont des vues de dessus schématiques d'un poignet gauche comportant des implants selon l'invention ;
- la figure 10D est une vue en perspective de l'articulation naturelle entre le métacarpe du pouce de la main gauche et le trapèze du poignet gauche ;
- la figure 10E est une vue en coupe selon le plan AA' de la figure 10D de l'articulation naturelle entre le métacarpe du pouce de la main gauche et le trapèze du poignet gauche ;
- la figure 10F est une vue en coupe selon le plan BB' de la figure 10D de l'articulation naturelle entre le métacarpe du pouce de la main gauche et le trapèze du poignet gauche ;
- les figures 10G à 10I montrent, respectivement en perspective, en coupe selon le plan AA' précité et en coupe selon le plan BB' précité, comment peut être introduit l'implant selon le second mode de réalisation de l'invention entre le métacarpe du pouce de la main gauche et le trapèze du poignet gauche ;
- la figure 11A est une vue de dessus schématique d'une main droite dans laquelle a été introduite une prothèse totale de poignet selon l'invention ;
- la figure 11B est une vue de dessus schématique d'une main droite dans laquelle a été introduite une prothèse partielle de poignet selon l'invention ;
- la figure 12 est une vue de dessus schématique d'un pied droit dans lequel ont été introduits des implants selon l'invention ;
- la figure 13 est une vue en perspective schématique d'une prothèse de cheville selon l'invention introduite dans un patient ; et
- la figure 14 est une vue de côté schématique d'une prothèse de disque intervertébral introduite dans un patient.

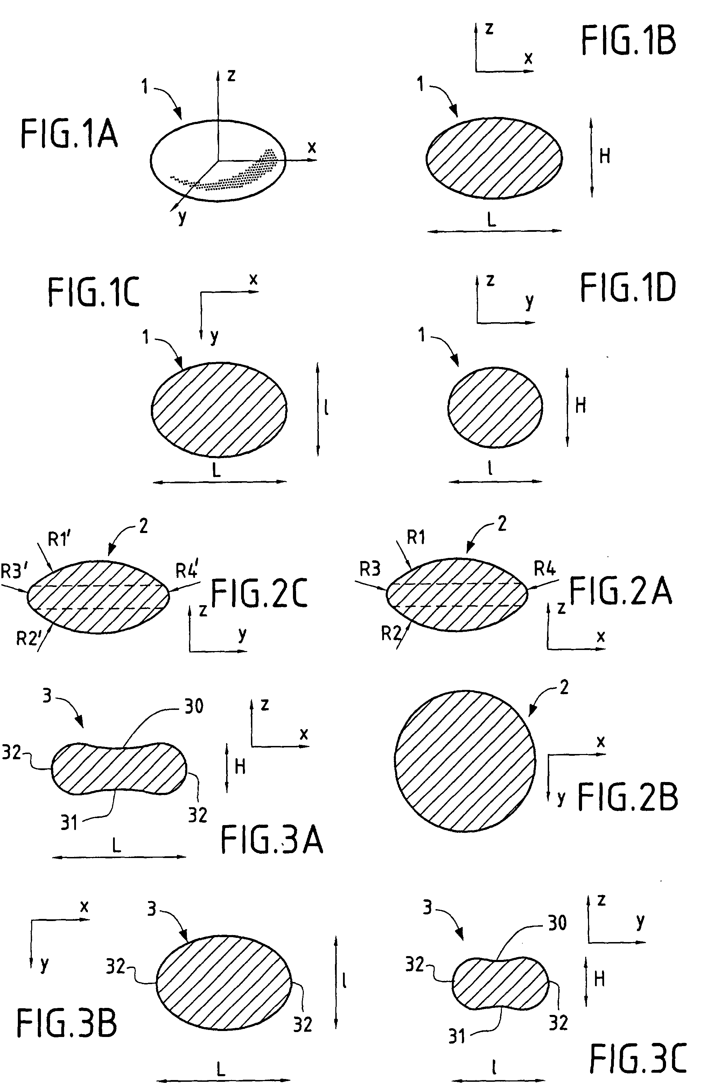

Les sections planes de l'implant qui sont représentées aux figures 1 à 8, et définies dans les revendications annexées à cette description, sont chacune obtenues par intersection de l'implant avec un plan passant par son centre géométrique, c'est-à-dire son centre de gravité lorsque sa masse est parfaitement homogène. De manière générale, dans la description de l'implant faite en référence aux figures 1 à 8, et dans les revendications, le terme "plan" se rapportera, même lorsque cela ne sera pas précisé, à un plan géométrique passant par le centre géométrique de l'implant.

Selon un premier mode de réalisation, représenté aux figures 1A, 1B, 1C et 1D, l'implant, désigné par le repère 1, est ellipsoïdal, c'est-à-dire qu'il présente une forme elliptique dans trois plans mutuellement perpendiculaires (x,z), (x,y) and (y,z). La longueur L, la largeur 1 et la hauteur H de l'implant sont déterminés dans chaque cas en fonction de l'articulation ou de l'os à remplacer.

Selon un second mode de réalisation, représenté aux figures 2A à 2C, l'implant, désigné par le repère 2, présente une première section plane ovale dans le plan (x,z) (figure 2A), une seconde section plane ovale dans le plan (y,z) (figure 2C) et une section plane en forme de disque dans le plan (x,y) (figure 2B).

Le contour de la première section plane ovale est constitué d'arcs de cercle, et plus particulièrement d'une première paire d'arcs de cercle opposés ayant des rayons de courbure respectifs R1 et R2, et d'une seconde paire d'arcs de cercle opposés ayant des rayons de courbure respectifs R3 et R4, les rayons R1 et R2 étant supérieurs aux rayons R3 et R4. Dans l'exemple illustré aux figures 2A à 2C, les rayons R1 et R2 sont égaux, de même que les rayons R3 et R4.

Le contour de la seconde section plane ovale est constitué d'arcs de cercle, et plus particulièrement d'une première paire d'arcs de cercle opposés ayant des rayons de courbure respectifs R1' et R2', et d'une seconde paire d'arcs de cercle opposés ayant des rayons de courbure respectifs R3' et R4', les rayons R1' et R2' étant supérieurs aux rayons R3' et R4'. Dans l'exemple illustré aux figures 2A à 2C, les rayons R1' et R2' sont égaux, de même que les rayons R3' et R4'.

Selon une variante préférée de l'implant 2, celui-ci est de révolution autour de l'axe z. En d'autres termes, les rayons de courbure R1 à R4 sont respectivement égaux aux rayons de courbure R1' à R4', et l'implant 2 présente la même forme dans tout plan perpendiculaire à la section en forme de disque et passant par le centre géométrique de l'implant, à savoir la forme des première et seconde sections planes ovales telle qu'illustrée aux figures 2A et 2C.

Selon un troisième mode de réalisation, représenté aux figures 3A, 3B et 3C, l'implant, désigné par le repère 3, comporte une première partie de surface 30 dont l'intersection avec chacun des plans perpendiculaires (x,z) et (y,z) est concave, et une seconde partie de surface 31, opposée à la première partie 30, dont l'intersection avec chacun des plans perpendiculaires (x,z) et (y,z) est également concave. L'implant 3 comprend en outre deux parties de surface latérales 32, de préférence sensiblement arrondies, reliant entre elles les parties de surface 30 et 31. En section plane dans le plan (x,y), l'implant 3 présente une forme ovale ou sensiblement elliptique. Par "sensiblement elliptique", on entend que cette section plane peut être elliptique, quasiment elliptique ou circulaire.

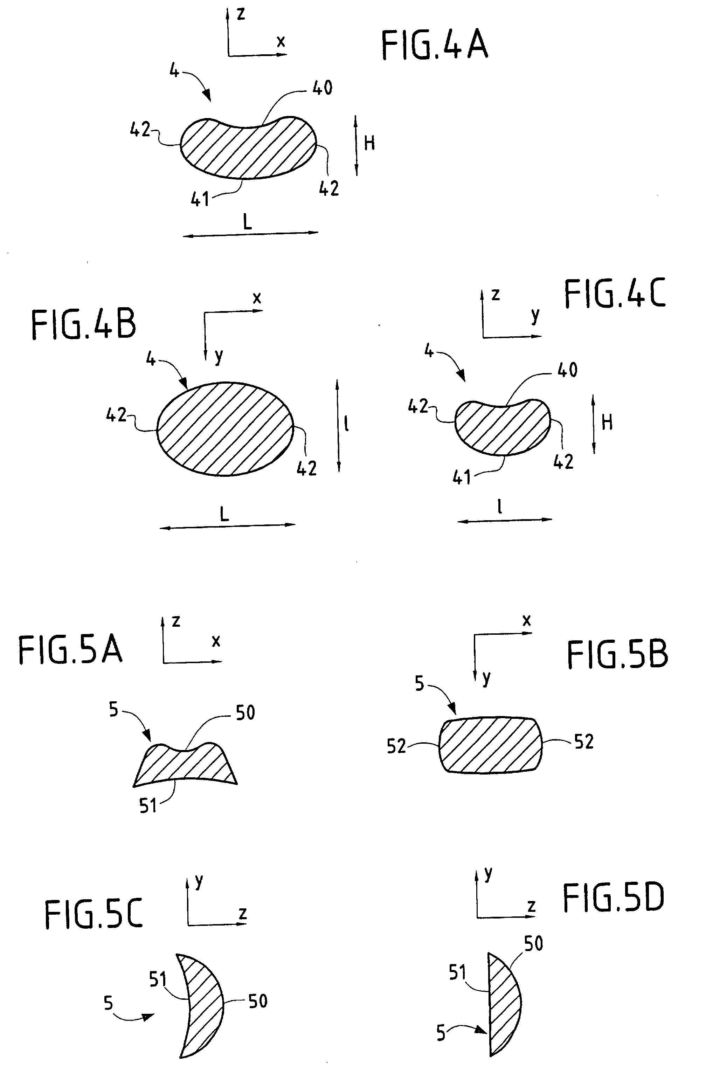

Selon un quatrième mode de réalisation, représenté aux figures 4A, 4B et 4C, l'implant, désigné par le repère 4, comporte une première partie de surface 40 dont l'intersection avec chacun des plans perpendiculaires (x,z) et (y,z) est concave, et une seconde partie de surface 41, opposée à la première partie 40, dont l'intersection avec chacun des plans perpendiculaires (x,z) et (y,z) est convexe. Les sections planes dans les plans (x,z) et (y,z) sont ainsi en forme de haricot ou de croissant. La section plane dans le plan (x,y) est ovale ou sensiblement elliptique. L'implant 4 comprend en outre des parties de surface latérales 42, de préférence sensiblement arrondies, reliant les parties de surface 40 et 41.

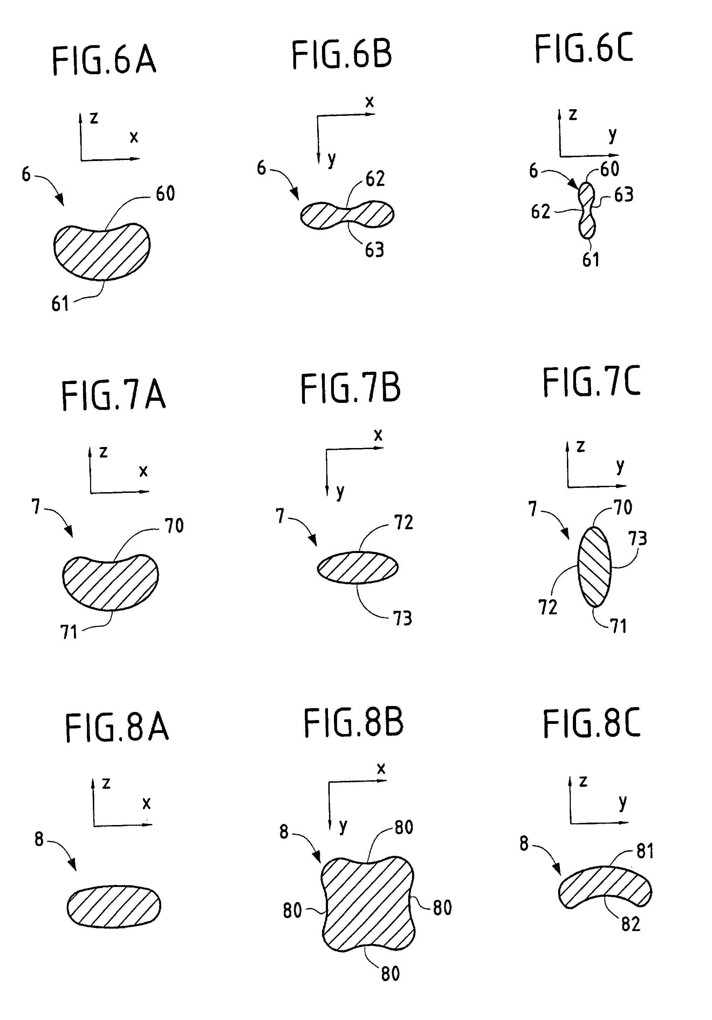

Selon un cinquième mode de réalisation, représenté aux figures 5A, 5B et 5C, l'implant, désigné par le repère 5, comporte une partie de surface 50 dont l'intersection avec le plan (x,z) est concave et l'intersection avec le plan (y,z) est convexe. L'implant 5 comprend en outre une partie de surface 51, opposée à la partie de surface 50, et dont l'intersection avec chacun des plans (x,z) et (y,z) est concave. En section dans le plan (y,z), l'implant 5 présente une forme de croissant de lune (figure 5C). Plus précisément, la section plane de l'implant 5 dans le plan (y,z) présente un contour constitué de deux parties de contour, l'une convexe, correspondant à la partie de surface 50, et l'autre concave, correspondant à la partie de surface 51. Chaque extrémité de la partie de contour convexe est confondue avec une extrémité correspondante de la partie de contour concave. La section dans le plan (x,y) est sensiblement rectangulaire, avec des bords latéraux 52 légèrement arrondis (figure 5B).

Selon une variante simplifiée de l'implant 5, la partie de surface 51 présente une section droite dans le plan (y,z), comme illustré à la figure 5D. Dans la variante de la figure 5D, la section plane de l'implant 5 dans le plan (y,z) présente un contour constitué de deux parties de contour, l'une convexe correspondant à la partie de surface 50, et l'autre droite correspondant à la partie de surface 51. Chaque extrémité de la partie de contour convexe est confondue avec une extrémité correspondante de la partie de contour droite.

Selon un sixième mode de réalisation, représenté aux figures 6A, 6B et 6C, l'implant, désigné par le repère 6, comporte une partie de surface 60, dont l'intersection avec le plan (x,z) est concave et l'intersection avec le plan (y,z) est convexe, et une partie de surface 61, opposée à la partie de surface 60, dont l'intersection avec chacun des plans (x,z) et (y,z) est convexe. L'implant 6 comporte en outre des parties de surface opposées 62 et 63 reliant entre elles les parties de surface 60 et 61. Les parties de surface 62, 63 ont chacune une intersection de forme concave avec les plans (x,y) et (y,z). Les parties de surface 62, 63 ont une plus grande taille que les parties de surface 60, 61, de sorte que l'implant 6 présente une forme aplatie dans les plans (x,y) et (y,z).

Selon un septième mode de réalisation, représenté aux figures 7A, 7B et 7C, l'implant, désigné par le repère 7, comporte une partie de surface 70, dont l'intersection avec le plan (x,z) est concave et l'intersection avec le plan (y,z) est convexe, et une partie de surface 71, opposée à la partie de surface 70, dont l'intersection avec chacun des plans (x,z) et (y,z) est convexe. L'implant 7 comporte en outre des parties de surface opposées 72 et 73 reliant entre elles les parties de surface 70 et 71. Les parties de surface 72, 73 ont chacune une intersection de forme convexe avec les plans (x,y) et (y,z). Les parties de surface 72, 73 ont une plus grande taille que les parties de surface 70, 71, de sorte que l'implant 7 présente une forme aplatie dans les plans (x,y) et (y,z).

Selon un huitième mode de réalisation, représenté aux figures 8A, 8B et 8C, l'implant, désigné par le repère 8, a une section plane sensiblement ovale dans le plan (x,z), sensiblement carrée ou rectangulaire dans le plan (x,y), et en forme de croissant dans le plan (y,z). Les côtés 80 de la section sensiblement carrée sont légèrement concaves, comme montré à la figure 8B. La section dans le plan (y,z) comporte une partie de contour convexe 81 opposée à une partie de contour concave 82.

Les implants 1 à 8 décrits ci-dessus, ou au moins leur surface externe, sont conçus en pyrocarbone. Afin de réduire encore davantage le coefficient de frottement entre l'implant et les os avoisinants, leur surface externe est en outre entièrement polie. Toute la surface externe de chaque implant 1 à 8 est lisse, continue et régulière, et ne comporte ainsi aucun moyen de fixation.

Les implants 1 à 8 sont fabriqués par exemple en dessinant la forme de l'implant par CAO (Conception Assistée par Ordinateur), en reproduisant cette forme sur un substrat en graphite à l'aide d'une machine fraiseuse à commande numérique reliée à l'ordinateur, puis en recouvrant le substrat en graphite d'une couche de pyrocarbone par une technique de dépôt en phase vapeur chimique CVD (Chemical Vapour Deposition) en lit fluidisé.

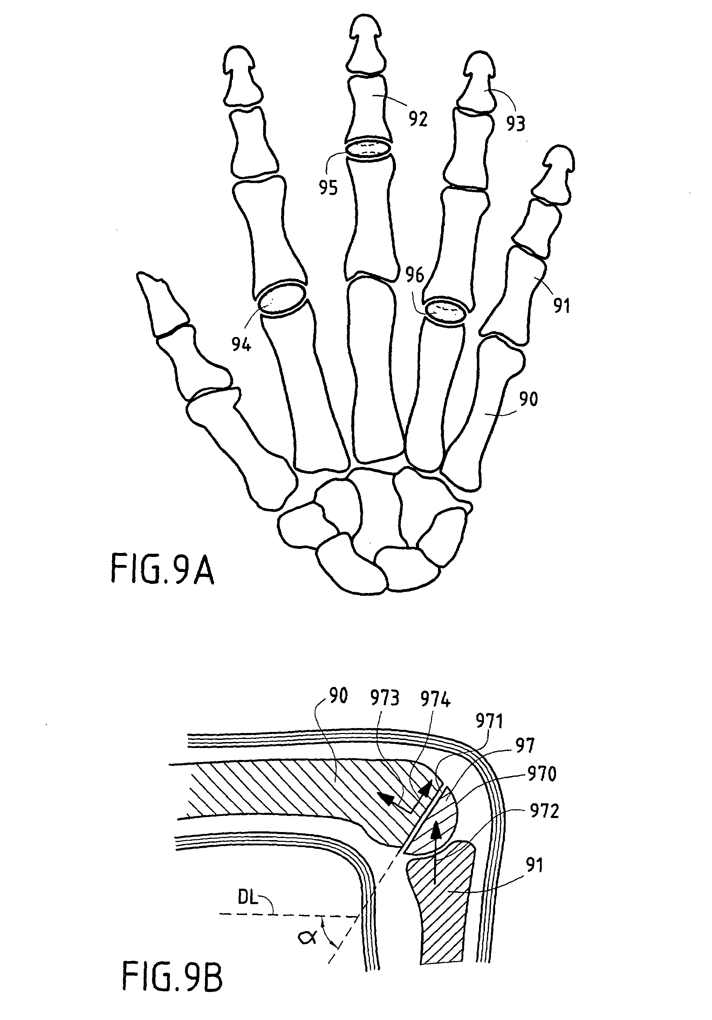

Les figures 9 à 14 montrent des exemples d'applications pour les implants 1 à 8 décrits ci-dessus.

La figure 9A représente une main droite en vue de dessus. Les implants 1 à 4 décrits ci-dessus peuvent chacun être implantés dans un doigt du patient pour servir d'articulation métacarpo-phalangienne, entre un métacarpe 90 et une phalange proximale 91, ou d'articulation interphalangienne, entre une phalange proximale 91 et une phalange moyenne 92, ou entre une phalange moyenne 92 et une phalange distale 93.

Le repère 94 désigne un implant selon l'invention, positionné entre le métacarpe et la phalange proximale de l'index. L'implant 94 est identique à l'implant 1 ou à l'implant 2 illustrés aux figures 1A à 1D et 2A, 2B. Le repère 95 désigne un implant selon l'invention positionné entre la phalange proximale et la phalange moyenne du majeur. L'implant 95 est identique à l'implant 3 illustré aux figures 3A à 3C. Le repère 96 désigne un implant selon l'invention, positionné entre le métacarpe et la phalange proximale de l'annulaire. L'implant 96 est identique à l'implant 4 illustré aux figures 4A à 4C.

Chacun des implants 94, 95, 96 est placé, sans être fixé, dans un logement de forme adaptée, ménagé préalablement à l'introduction de l'implant. Ainsi, le logement de l'implant 94, entre le métacarpe et la phalange proximale de l'index, présente une forme sensiblement ovoïdale ou ellipsoïdale, ou éventuellement rectangulaire, tandis que le logement de l'implant 95 comporte par exemple deux faces concaves en regard et le logement de l'implant 96 comporte par exemple une face concave et une face convexe en regard l'une de l'autre.

La figure 9B représente, en section plane, un implant d'articulation métacarpo-phalangienne 97 positionné entre le métacarpe 90 et la phalange proximale 91 d'un doigt. Dans la configuration illustrée à la figure 9B, le doigt est replié. L'implant 97 est du type de l'implant 5 illustré aux figures 5A à 5D, et comporte une surface 970, correspondant à la surface 50 des figures 5A à 5D, faisant face à la phalange 91 et une surface 971, correspondant à la surface 51, en regard du métacarpe 90. Grâce à la forme de l'implant 97, une partie des contraintes exercées par la phalange 91 sur l'implant 97 dans le sens de la flèche 972 sont transmises au métacarpe 90, comme indiqué par la flèche 973, par le fait que l'implant 97 s'appuie sur le métacarpe 90, tandis qu'une autre partie (flèche 974) est absorbée par les ligaments et tendons avoisinants. Ainsi, la forme de l'implant 97 permet d'éviter que celui-ci ne soit éjecté de son logement. Le logement de l'implant 97 est obtenu en retirant une partie de l'extrémité du métacarpe 90 de telle façon que la surface de l'extrémité résultante fasse un angle a avec une direction longitudinale DL du métacarpe 90.

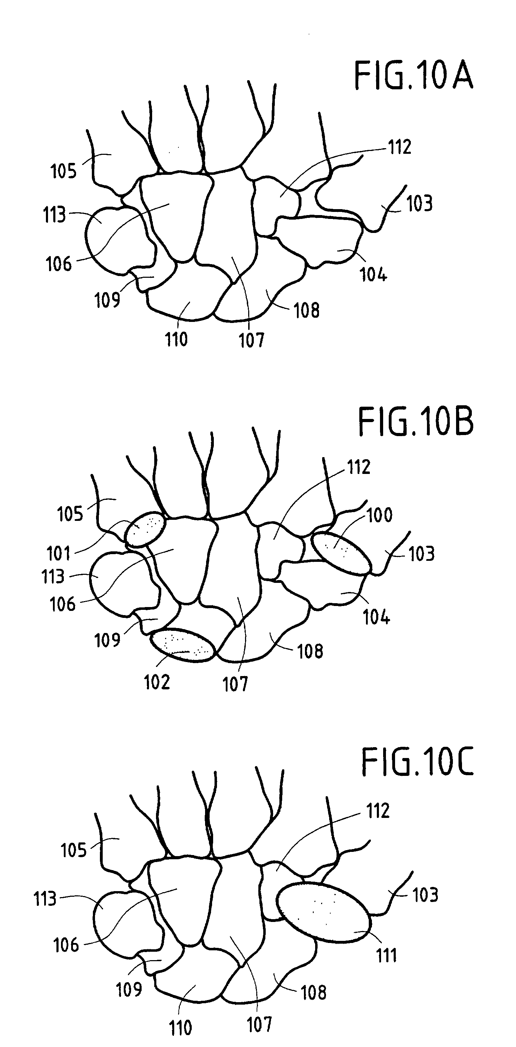

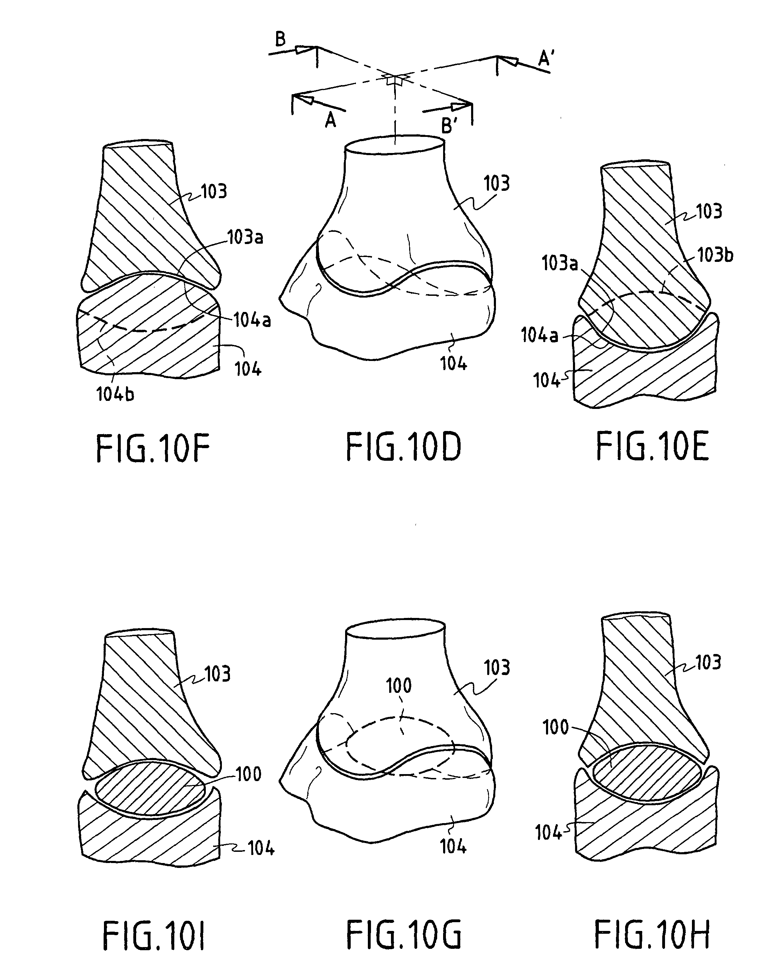

La figure 10A représente un poignet gauche, vu de dessus, et la figure 10B représente ce même poignet gauche dans laquelle des implants 100, 101 et 102 selon l'invention ont été introduits. L'implant 100 sert d'articulation trapézo-métacarpienne. Cette implant est ainsi placé dans un logement obtenu en rognant les surfaces en regard du métacarpe 103 du pouce et du trapèze 104. L'implant 101 sert d'articulation carpo-métacarpienne entre notamment le métacarpe 105 de l'auriculaire et l'os crochu 106. L'implant 102 sert d'articulation radio-carpienne entre le radius (non représenté) et le grand os 107, et est en contact avec le scaphoïde 108 et l'os pyramidal 109, pour jouer le rôle de clé de voûte du poignet en remplacement du semi-lunaire 110. Les implants 100, 101 et 102 sont de préférence identiques à l'implant 1 ou à l'implant 2 illustrés aux figures 1A à 1D et 2A, 2B.

La figure 10C représente le poignet gauche de la figure 10A avec une prothèse de trapèze 111. Le logement de la prothèse 111 est préparé en retirant le trapèze 104 et en rognant légèrement la surface du métacarpe 103 en regard du trapèze 104. La prothèse 111 est également identique à l'implant 1 ou 2.

Les figures 10D à 10I montrent plus particulièrement comment est introduit l'implant 100 illustré à la figure 10B lorsque celui-ci est du type de l'implant 2 représenté aux figures 2A, 2B.

Les figures 10D, 10E et 10F représentent respectivement en perspective et en coupe suivant les plans AA' et BB' de la figure 10D, l'articulation naturelle entre le métacarpe 103 du pouce et le trapèze 104. Cette articulation se présente, de manière générale, sous la forme d'une double selle de cheval. Comme montré aux figures 10E et 10F, la surface d'articulation du métacarpe 103 comporte une partie de surface centrale 103a qui est convexe dans le plan AA' et concave dans le plan BB'. La surface d'articulation du trapèze 104 comporte une partie de surface centrale 104a qui est concave dans le plan AA' et convexe dans le plan BB'.

Afin de constituer un logement pour l'implant 100, la partie centrale 103a de la surface d'articulation du métacarpe 103 est rognée jusqu'au trait pointillé 103b représenté à la figure 10E, de sorte que cette partie centrale devienne concave dans le plan AA', mais reste concave dans le plan BB'. De façon comparable, la partie centrale 104a de la surface d'articulation du trapèze 104 est rognée jusqu'au trait pointillé 104b représenté à la figure 10F, de sorte que cette partie centrale devienne concave dans le plan BB', mais reste concave dans le plan AA'.

De cette manière, un logement est formé pour l'implant 100 qui respecte bien la forme globale des surfaces articulaires, puisque seule une partie centrale de chacune de ces surfaces est retirée. En outre, en prenant comme rayons de courbure R1, R2 et R1', R2' de l'implant (cf. figures 2A et 2C) le rayon de courbure des surfaces articulaires rognées (cf. figures 10I et 10H), on conserve la cinématique d'origine de l'articulation (cf. figures 10E et 10F), le centre de rotation du déplacement du métacarpe 103 par rapport à la surface articulaire du trapèze 104 restant inchangé.

D'autre part, la forme circulaire de l'implant 100 en vue de dessus (cf. figure 2B) garantit que l'implant remplira bien l'espace à l'intérieur de son logement et restera donc stable, tout en étant sensiblement mobile. Le fait, selon une variante préférée du second mode de réalisation de l'invention, que l'implant ait la même forme ovale dans tout plan perpendiculaire au plan de sa section plane en forme de disque et passant par le centre géométrique de l'implant, et qu'il soit donc parfaitement symétrique, favorise la mobilité de l'implant 100 dans son logement, puisque ledit implant n'a pas besoin d'être orienté d'une manière particulière. L'implant 100 peut ainsi tourner aléatoirement sur lui-même dans le plan de sa section plane en forme de disque lors de mouvements variés effectués par le patient. Une zone donnée de la surface articulaire du métacarpe 103 ou du trapèze 104 verra donc, au fil des mouvements du patient, des zones différentes de la surface de l'implant 100, sans que la cinématique de l'articulation n'en soit affectée. Grâce aux mouvements aléatoires de l'implant 100, on empêche qu'un phénomène de cicatrisation naturelle se produise entre l'implant et les tissus ligamentaires et/ou osseux environnants.

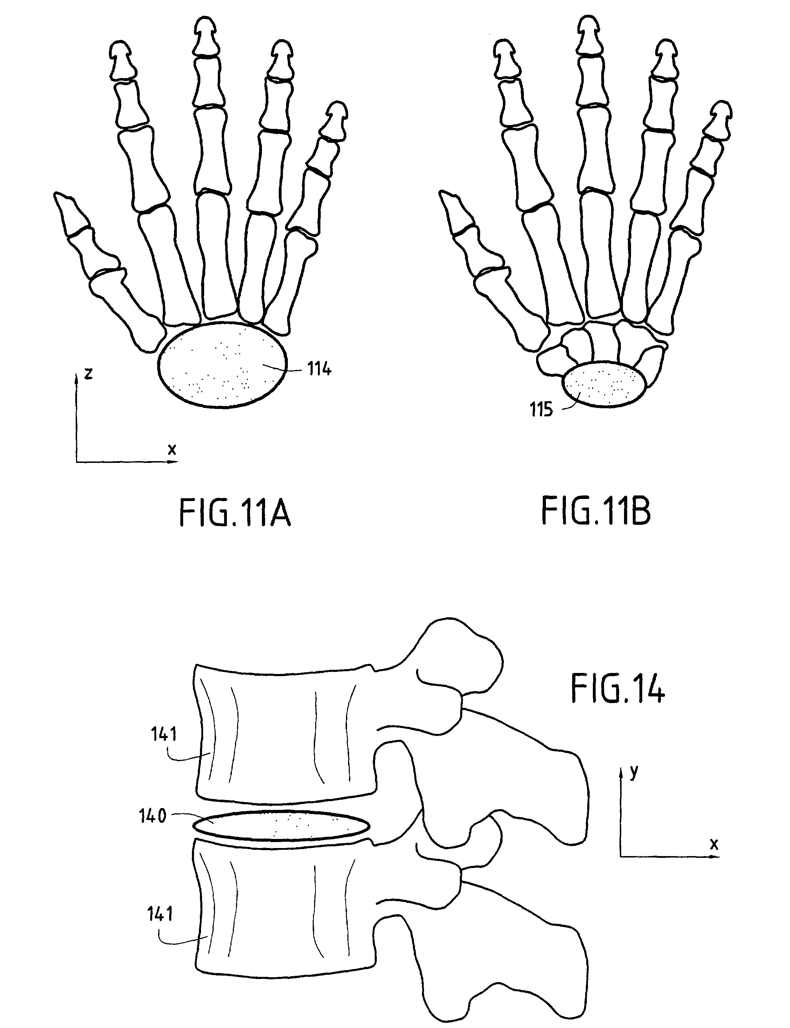

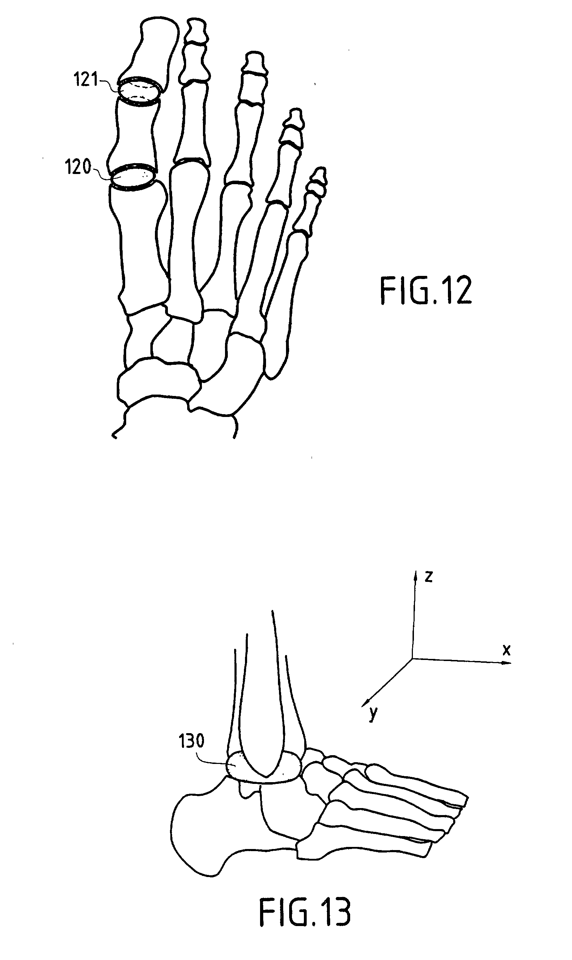

La figure 11A représente une prothèse totale de poignet 114 selon l'invention, du type de l'implant 1 ou 2, remplaçant tous les os du poignet, à savoir le trapèze 104, le trapézoïde 112, le scaphoïde 108, le semi-lunaire 110, l'os pyramidal 109, l'os pisiforme 113, le grand os 107 et l'os crochu 106. La figure 11B représente une prothèse partielle de poignet 115 selon l'invention, remplaçant la première rangée du carpe, à savoir le scaphoïde 108, l'os pyramidal 109 et le semi-lunaire 110.

La figure 12 représente un pied droit en vue de dessus, comprenant un implant d'articulation métatarso-phalangienne 120 et un implant d'articulation interphalangienne 121, qui peuvent chacun être constitués par l'un des implants 1 à 4 précédemment décrits.

La figure 13 montre une prothèse de cheville 130 selon l'invention, implantée dans le patient, sans être fixée, en remplacement de l'astragale. La prothèse 130 est de préférence identique à l'implant 8 illustré aux figures 8A à 8C, mais peut également consister en l'un des implants 1 et 2 des figures 1A à 1D et 2A, 2B.

La figure 14 représente en vue de côté une prothèse de disque intervertébral 140 selon l'invention, placée entre deux vertèbres 141. La prothèse 140 est identique à l'implant 6 illustré aux figures 6A à 6C, ou à l'implant 7 illustré aux figures 7A à 7C.

## Revendications

1. Implant destiné à servir de pièce d'interposition entre des surfaces osseuses d'un patient, **caractérisé en ce qu'**au moins sa surface externe comprend du pyrocarbone, et **en ce qu'**il est dépourvu de tout moyen de fixation, de telle sorte que l'implant reste libre par rapport auxdites surfaces osseuses lorsqu'il est introduit dans le patient.

2. Implant selon la revendication 1, **caractérisé en ce qu'**il est conçu en un matériau dont le module d'élasticité est approximativement compris entre 10 et 35 GPa.

3. Implant selon la revendication 1 ou 2, **caractérisé en ce qu'**au moins sa surface externe est conçue en pyrocarbone.

4. Implant selon l'une quelconque des revendications 1 à 3, **caractérisé en ce que** sa surface externe est polie.

5. Implant (1) selon l'une quelconque des revendications 1 à 4, **caractérisé en ce qu'**il présente une forme sensiblement ellipsoïdale ou ovoïdale.

6. Implant (2) selon l'une quelconque des revendications 1 à 4, **caractérisé en ce qu'**il présente une première section plane ovale et une seconde section plane ovale perpendiculaires l'une à l'autre, et une section plane en forme de disque, perpendiculaire aux première et seconde sections planes ovales.

7. Implant (2) selon la revendication 6, **caractérisé en ce que** chacune des première et seconde sections planes ovales comporte un contour constitué d'arcs de cercle.

8. Implant (2) selon la revendication 6 ou 7, **caractérisé en ce que** les première et seconde sections planes ovales ont la même forme, et l'implant présente, dans tout plan perpendiculaire à la section plane en forme de disque et passant par le centre géométrique de l'implant, la forme des première et seconde sections planes ovales.

9. Implant (3) selon l'une quelconque des revendications 1 à 4, **caractérisé en ce qu'**il comporte une première partie de surface (30) dont les intersections respectives avec un premier plan (x,z) et un second plan (y,z), perpendiculaire au premier plan, sont concaves, et une seconde partie de surface (31), opposée à la première partie de surface (30), dont les intersections respectives avec les premier et second plans sont également concaves.

10. Implant (4) selon l'une quelconque des revendications 1 à 4, **caractérisé en ce qu'**il comprend une première partie de surface (40) dont les intersections respectives avec un premier plan (x,z) et un second plan (y,z), perpendiculaire au premier plan, sont concaves, et une seconde partie de surface (41), opposée à la première partie de surface (40), dont les intersections respectives avec les premier et second plans sont convexes.

11. Implant (3; 4) selon la revendication 9 ou 10, **caractérisé en ce qu'**il présente en outre une section plane, dans un troisième plan (x,y) perpendiculaire aux premier et second plans, de forme sensiblement elliptique.

12. Implant (5) selon l'une quelconque des revendications 1 à 4, **caractérisé en ce qu'**il présente une section plane dont le contour est constitué d'une partie convexe (50) et d'une partie concave (51), chaque extrémité de la partie convexe étant confondue avec une extrémité correspondante de la partie concave.

13. Implant (5) selon l'une quelconque des revendications 1 à 4, **caractérisé en ce qu'**il présente une section plane dont le contour est constitué d'une partie convexe (50) et d'une partie sensiblement droite (51), chaque extrémité de la partie convexe étant confondue avec une extrémité correspondante de la partie sensiblement droite.

14. Implant (5) selon la revendication 12 ou 13, **caractérisé en ce qu'**il comporte une partie de surface (50) dont l'intersection avec un premier plan (x,z) est concave, et l'intersection avec un second plan (y,z), perpendiculaire au premier plan, constitue ladite partie convexe.

15. Implant (5) selon la revendication 12, **caractérisé en ce qu'**il comporte une première partie de surface (50) dont l'intersection avec un premier plan (x,z) est concave, et l'intersection avec un second plan (y,z), perpendiculaire au premier plan, constitue ladite partie convexe, et une seconde partie de surface (51) dont l'intersection avec le premier plan est concave et l'intersection avec le second plan constitue ladite partie concave.

16. Implant (5) selon la revendication 13, **caractérisé en ce qu'**il comporte une première partie de surface (50) dont l'intersection avec un premier plan (x,z) est concave, et l'intersection avec un second plan (y,z), perpendiculaire au premier plan, constitue ladite partie convexe, et une seconde partie de surface (51) dont l'intersection avec le premier plan est concave et l'intersection avec le second plan constitue ladite partie sensiblement droite.

17. Implant (5) selon la revendication 14, 15 ou 16, **caractérisé en ce qu'**il présente une section plane dans un troisième plan, perpendiculaire aux premier et second plans, de forme sensiblement rectangulaire avec de préférence des bords latéraux arrondis (52).

18. Implant (6 ; 7) selon l'une quelconque des revendications 1 à 4, **caractérisé en ce qu'**il comporte une première partie de surface (60 ; 70) dont l'intersection avec un premier plan (x,z) est concave et l'intersection avec un second plan (y,z), perpendiculaire au premier plan, est convexe, et une seconde partie de surface (61 ; 71), opposée à la première partie de surface (60 ; 70), dont les intersections respectives avec les premier et second plans sont convexes.

19. Implant (6) selon la revendication 18, **caractérisé en ce qu'**il comporte en outre une troisième partie de surface (62) dont les intersections respectives avec le second plan (y,z) et un troisième plan (x,y), perpendiculaire aux premier et second plans, sont concaves, et une quatrième partie de surface (63), opposée à la troisième partie de surface (62), dont les intersections respectives avec les second et troisième plans sont concaves, les troisième et quatrième parties de surface reliant les première et seconde parties de surface entre elles.

20. Implant (7) selon la revendication 18, **caractérisé en ce qu'**il comporte en outre une troisième partie de surface (72) dont les intersections respectives avec le second plan (y,z) et un troisième plan (x,y), perpendiculaire aux premier et second plans, sont convexes, et une quatrième partie de surface (73), opposée à la troisième partie de surface (72), dont les intersections respectives avec les second et troisième plans sont convexes, les troisième et quatrième parties de surface reliant les première et seconde parties de surface entre elles.

21. Implant (6 ; 7) selon la revendication 19 ou 20, **caractérisé en ce qu'**il présente une forme aplatie dans les second et troisième plans.

22. Implant (8) selon l'une quelconque des revendications 1 à 4, **caractérisé en ce qu'**il présente une première section plane sensiblement ovale et une seconde section plane, perpendiculaire à la première section plane, comportant une partie de contour convexe (81) et une partie de contour concave (82) opposée à la partie convexe (81).

23. Implant (8) selon la revendication 22, **caractérisé en ce qu'**il présente en outre une troisième section plane, perpendiculaire aux première et seconde sections planes, de forme sensiblement carrée ou rectangulaire et dont les côtés sont de préférence concaves.

24. Implant selon l'une quelconque des revendications 1 à 4, **caractérisé en ce qu'**il a une forme et une taille adaptées pour pouvoir être utilisé en tant que l'une des articulations suivantes : articulation interphalangienne, articulation métacarpo-phalangienne, articulation trapézo-métacarpienne, articulation radio-carpienne, articulation carpo-métacarpienne, et articulation métatarso-phalangienne.

25. Implant selon l'une quelconque des revendications 1 à 4, **caractérisé en ce qu'**il constitue une prothèse de l'un des types suivants : prothèse de cheville, prothèse de disque intervertébral, prothèse totale de poignet, et prothèse partielle de poignet.

## Patentansprüche

1. Implantat, dafür bestimmt, als Einsatzstück zwischen Knochenoberflächen eines Patienten zu dienen, **dadurch gekennzeichnet, dass** zumindest seine Aussenfläche Pyrokohlenstoff umfasst und dass es frei von jeglichen Befestigungsmitteln ist, so dass das Implantat gegenüber diesen Knochenoberflächen frei bleibt, wenn es in den Patienten eingeführt worden ist.

2. Implantat gemäss Anspruch 1, **dadurch gekennzeichnet, dass** es aus einem Material konzipiert ist, dessen Elastizitätsmodul ungefähr zwischen 10 und 35 GPa liegt.

3. Implantat gemäss Anspruch 1 oder 2, **dadurch gekennzeichnet, dass** zumindest seine Aussenfläche aus Pyrokohlenstoff konzipiert ist.

4. Implantat gemäss einem beliebigen der Ansprüche 1 bis 3, **dadurch gekennzeichnet, dass** seine Aussenfläche poliert ist.

5. Implantat (1) gemäss einem beliebigen der Ansprüche 1 bis 4, **dadurch gekennzeichnet, dass** es eine im Wesentlichen ellipsoidische oder ovoide Gestalt aufweist.

6. Implantat (2) gemäss einem beliebigen der Ansprüche 1 bis 4, **dadurch gekennzeichnet, dass** es einen ovalen ersten ebenen Schnitt und einen ovalen zweiten ebenen Schnitt, die zueinander senkrecht sind, sowie einen scheibenförmigen ebenen Schnitt aufweist, der senkrecht zum ersten und zweiten ovalen ebenen Schnitt ist.

7. Implantat (2) gemäss Anspruch 6, **dadurch gekennzeichnet, dass** der erste und der zweite ebene ovale Schnitt je eine aus Kreisbögen zusammengesetzte Kontour umfassen.

8. Implantat (2) gemäss Anspruch 6 oder 7, **dadurch gekennzeichnet, dass** der erste und der zweite ebene ovale Schnitt die gleiche Gestalt haben und dass das Implantat in jeder Ebene, die zum scheibenförmigen ebenen Schnitt senkrecht verläuft und durch das geometrische Zentrum des Implantats hindurchgeht, die Gestalt des ovalen ebenen ersten und zweiten Schnitts aufweist.

9. Implantat (3) gemäss einem beliebigen der Ansprüche 1 bis 4, **dadurch gekennzeichnet, dass** es einen ersten Oberflächenabschnitt (30) umfasst, dessen Schnittstellen mit einer ersten Ebene (x, z) und mit einer zur ersten Ebene senkrechten zweiten Ebene (y, z) konkav sind, sowie einen dem ersten Oberflächenabschnitt (30) entgegengesetzten zweiten Oberflächenabschnitt (31), dessen Schnittstellen mit der ersten und zweiten Ebene ebenfalls konkav sind.

10. Implantat (4) gemäss einem beliebigen der Ansprüche 1 bis 4, **dadurch gekennzeichnet, dass** es einen ersten Oberflächenabschnitt (40) umfasst, dessen Schnittstellen mit einer ersten Ebene (x, z) und mit einer zur ersten Ebene senkrechten zweiten Ebene (y, z) konkav sind, sowie einen dem ersten Oberflächenabschnitt (40) entgegengesetzten zweiten Oberflächenabschnitt (41), dessen Schnittstellen mit der ersten und zweiten Ebene konvex sind.

11. Implantat (3; 4) gemäss Anspruch 9 oder 10, **dadurch gekennzeichnet, dass** es ausserdem einen ebenen Schnitt von im Wesentlichen elliptischer Gestalt in einer zur ersten und zweiten Ebene senkrechten dritten Ebene (x, y) aufweist.

12. Implantat (5) gemäss einem beliebigen der Ansprüche 1 bis 4, **dadurch gekennzeichnet, dass** es einen ebenen Schnitt aufweist, dessen Kontur aus einem konvexen Abschnitt (50) und einem konkaven Abschnitt (51) besteht, wobei jedes Ende des konvexen Abschnitts mit einem entsprechenden Ende des konkaven Abschnitts zusammenfliesst.

13. Implantat (5) gemäss einem beliebigen der Ansprüche 1 bis 4, **dadurch gekennzeichnet, dass** es einen ebenen Schnitt aufweist, dessen Kontur aus einem konvexen Abschnitt (50) und einem im Wesentlichen geraden Abschnitt (51) besteht, wobei jedes Ende des konvexen Abschnitts mit einem entsprechenden Ende des im Wesentlichen geraden Abschnitts zusammenfliesst.

14. Implantat (5) gemäss Anspruch 12 oder 13, **dadurch gekennzeichnet, dass** es einen Oberflächenabschnitt (50) umfasst, dessen Schnittstelle mit einer ersten Ebene (x, z) konkav ist, und die Schnittstelle mit einer zur ersten Ebene senkrechten zweiten Ebene (y, z) den konvexen Abschnitt darstellt.

15. Implantat (5) gemäss Anspruch 12, **dadurch gekennzeichnet, dass** es einen ersten Oberflächenabschnitt (50) umfasst, dessen Schnittstelle mit einer ersten Ebenen (x, z) konkav ist, während seine Schnittstelle mit einer zur ersten Ebene senkrechten zweiten Ebene (y, z) den konvexen Abschnitt darstellt, sowie einen zweiter Oberflächenabschnitt (51), dessen Schnittstelle mit der ersten Ebene konkav ist, während seine Schnittstelle mit der zweiten Ebene den konkaven Abschnitt darstellt.

16. Implantat (5) gemäss Anspruch 13, **dadurch gekennzeichnet, dass** es einen ersten Oberflächenabschnitt (50) umfasst, dessen Schnittstelle mit einer ersten Ebene (x, z) konkav ist, während seine Schnittstelle mit einer zur ersten Ebene senkrechten zweiten Ebene (y, z) den konvexen Abschnitt darstellt, sowie einen zweiten Oberflächenabschnitt (51), dessen Schnittstelle mit der ersten Ebene konkav ist, während seine Schnittstelle mit der zweiten Ebene den im Wesentlichen geraden Abschnitt darstellt.

17. Implantat gemäss Anspruch 14, 15 oder 16, **dadurch gekennzeichnet, dass** es in einer zur ersten und zweiten Ebene senkrechten dritten Ebene einen ebenen Schnitt von im Wesentlichen rechteckiger Gestalt, bevorzugt mit abgerundeten seitlichen Rändern (52), aufweist.

18. Implantat (6; 7) gemäss einem beliebigen der Ansprüche 1 bis 4, **dadurch gekennzeichnet, dass** es einen ersten Oberflächenabschnitt (60; 70) umfasst, dessen Schnittstelle mit einer ersten Ebene (x, z) konkav ist, während seine Schnittstelle mit einer zur ersten Ebene senkrechten zweiten Ebene (y, z) konvex ist, sowie einen dem ersten Oberflächenabschnitt (60; 70) entgegengesetzten zweiten Oberflächenabschnitt (61, 71), dessen Schnittstellen mit der ersten bzw. zweiten Ebene konvex sind.

19. Implantat (6) gemäss Anspruch 18, **dadurch gekennzeichnet, dass** es ausserdem einen dritten Oberflächenabschnitt (62), dessen Schnittstellen mit der zweiten Ebene (y, z) und einer zur ersten und zweiten Ebene senkrechten dritten Ebene (x, y) konkav sind, sowie einen dem dritten Oberflächenabschnitt (62) entgegengesetzten vierten Oberflächenabschnitt (63) umfasst, dessen Schnittstellen mit der zweiten bzw, dritten Ebene konkav sind, wobei der dritte und vierte Oberflächenabschnitt den ersten und zweiten Oberflächenabschnitt miteinander verbinden.

20. Implantat (7) gemäss Anspruch 18, **dadurch gekennzeichnet, dass** es ausserdem einen dritten Oberflächenabschnitt (72), dessen Schnittstellen mit der zweiten Ebene (y, z) und einer zur ersten und zweiten Ebene senkrechten dritten Ebene (x, y) konvex sind, sowie einen dem dritten Oberflächenabschnitt (72) entgegengesetzten vierten Oberflächenabschnitt (73) umfasst, dessen Schnittstellen mit der zweiten bzw. dritten Ebene konvex sind, wobei der dritte und vierte Oberflächenabschnitt den ersten und zweiten Oberflächenabschnitt miteinander verbinden.

21. Implantat (6; 7) gemäss Anspruch 19 oder 20, **dadurch gekennzeichnet, dass** es in der zweiten und dritten Ebene eine abgeflachte Gestalt aufweist.

22. Implantat (8) gemäss einem beliebigen der Ansprüche 1 bis 4, **dadurch gekennzeichnet, dass** es einen im Wesentlichen ovalen ersten ebenen Schnitt sowie einen zum ersten ebenen Schnitt senkrechten zweiten ebenen Schnitt aufweist, der einen konvexen Konturabschnitt (81) und einen dem konvexen Abschnitt (81) entgegengesetzten konkaven Konturabschnitt (82) umfasst.

23. Implantat (8) gemäss Anspruch 22, **dadurch gekennzeichnet, dass** es ausserdem einen zum ersten und zweiten ebenen Schnitt senkrechten dritten ebenen Schnitt von im Wesentlichen quadratischer oder rechteckiger Gestalt aufweist, dessen Seiten bevorzugt konkav sind.

24. Implantat gemäss einem beliebigen der Ansprüche 1 bis 4, **dadurch gekennzeichnet, dass** es eine Gestalt und eine Grösse aufweist, die geeignet sind dass es als eine der folgenden Gelenke eingesetzt werden kann: Interphalangealgelenk, Metacarpophalangealgelenk, Daumensattelgelenk, Radiocarpalgelenk, Carpometacarpalgelenk und Metatarsophalangealgelenk.

25. Implantat gemäss einem beliebigen der Ansprüche 1 bis 4, **dadurch gekennzeichnet, dass** es eine Prothese eines der folgenden Typen darstellt: Knöchelprothese, Bandscheibenprothese, Handgelenkvollprothese und Handgelenkteilprothese.

## Claims

1. An implant intended for serving as an interposition piece between bony surfaces of a patient, **characterised in that** at least its external surface comprises pyrolytic carbon and **in that** it is free from any attaching means so that the implant remains free with respect to said bony surfaces when implanted in the patient.

2. An implant according to claim 1, **characterised in that** it is made of a material having a modulus of elasticity approximately comprised between 10 and 35 GPa.

3. An implant according to claim 1 or 2, **characterised in that** at least its external surface is made of pyrolytic carbon.

4. An implant according to any one of claims 1 to 3, **characterised in that** its external surface is polished.

5. An implant (1) according to any one of claims 1 to 4, **characterised in that** it has a substantially ellipsoidal or ovoid shape.

6. An implant (2) according to any one of claims 1 to 4, **characterised in that** it has a first oval plane section and a second oval plane section perpendicular to each other, and a disc-shape plane section perpendicular to the first and second oval plane sections.

7. An implant (2) according to claim 6, **characterised in that** each of the first and second oval plane sections includes a contour consisting of arcs of circles.

8. An implant (2) according to claim 6 or 7, **characterised in that** the first and second oval plane sections have the same shape and the implant has the shape of the first and second oval plane sections in any plane perpendicular to the disc-shape plane section and passing through the geometrical centre of the implant.

9. An implant (3) according to any one of claims 1 to 4, **characterised in that** it comprises a first surface portion (30) whose respective intersections with a first plane (x,z) and a second plane (y,z) perpendicular to the first plane are concave, and a second surface portion (31 ), opposite the first surface portion (30), whose respective intersections with the first and second planes are also concave.

10. An implant (4) according to any one of claims 1 to 4, **characterised in that** it comprises a first surface portion (40) whose respective intersections with a first plane (x,z) and a second plane (y,z) perpendicular to the first plane are concave, and a second surface portion (41), opposite the first surface portion (40), whose respective intersections with the first and second planes are convex.

11. An implant (3; 4) according to claim 9 or 10, **characterised in that** it further comprises a plane section, in a third plane (x,y) perpendicular to the first and second planes, with a substantially elliptic shape.

12. An implant (5) according to any one of claims 1 to 4, **characterised in that** it has a plane section with a contour consisting of a convex portion (50) and a concave portion (51), wherein each end of the convex portion joins a corresponding end of the concave portion.

13. An implant (5) according to any one of claims 1 to 4, **characterised in that** it has a plane section with a contour consisting of a convex portion (50) and a substantially straight portion (51), wherein each end of the convex portion joins a corresponding end of the substantially straight portion.

14. An implant (5) according to claim 12 or 13, **characterised in that** it comprises a surface portion (50) whose intersection with a first plane (x,z) is concave and whose intersection with a second plane (y,z) perpendicular to the first plane consists of said convex portion.

15. An implant (5) according to claim 12, **characterised in that** it comprises a first surface portion (50) whose intersection with a first plane (x,z) is concave and whose intersection with a second plane (y,z) perpendicular to the first plane consists of said convex portion, and a second surface portion (51) whose intersection with the first plane is concave and whose intersection with the second plane consists of said concave portion.

16. An implant (5) according to claim 13, **characterised in that** it comprises a first surface portion (50) whose intersection with a first plane (x,z) is concave and whose intersection with a second plane (y,z) perpendicular to the first plane consists of said convex portion, and a second surface portion (51) whose intersection with the first plane is concave and whose intersection with the second plane consists of said substantially straight portion.

17. An implant (5) according to claim 14, 15 or 16, **characterised in that** it comprises a plane section in a third plane perpendicular to the first and second planes, having a substantially rectangular shape with lateral edges that are preferably rounded.

18. An implant (6; 7) according to any one of claims 1 to 4, **characterised in that** it comprises a first surface portion (60; 70) whose intersection with a first plane (x,z) is concave and whose intersection with a second plane (y,z), perpendicular to the first plane, is convex, and a second surface portion (61; 71), opposite the first surface portion (60; 70), whose respective intersections with the first and second planes are convex.

19. An implant (6) according to claim 18, **characterised in that** it further comprises a third surface portion (62) whose respective intersections with the second plane (y,z) and a third plane (x,y), perpendicular to the first and second planes, are concave, and a fourth surface portion (63), opposite the third surface portion (62), whose respective intersections with the second and third planes are concave, wherein the third and fourth surface portions connect the first and second surface portions to each other.

20. An implant (7) according to claim 18, **characterised in that** it further comprises a third surface portion (72) whose respective intersections with the second plane (y,z) and a third plane (x,y), perpendicular to the first and second planes, are convex, and a fourth surface portion (73), opposite the third surface portion (72), whose respective intersections with the second and third planes are convex, wherein the third and fourth surface portions connect the first and second surface portions to each other.

21. An implant (6; 7) according to claim 19 or 20, **characterised in that** it has a flat shape in the second and third planes.

22. An implant (8) according to any one of claims 1 to 4, **characterised in that** it has a substantially oval first plane section and a second plane section perpendicular to the first plane section and having a convex contour portion (81) and a concave contour portion (82) opposite the convex portion (81).

23. An implant (8) according to claim 22, **characterised in that** it further comprises a third plane section, perpendicular to the first and second plane sections, having a substantially square or rectangular shape and whose sides are preferably concave.

24. An implant according to any one of claims 1 to 4, **characterised in that** it has a shape and a size adapted to be used as one of the following joints: an interphalangeal joint, a metacarpo-phalangeal joint, a trapezio-metacarpat joint, a radio-carpal joint, a carpo-metacarpal joint, and a metatarsophalangeal joint.

25. An implant according to any one of claims 1 to 4, **characterised in that** it consists of one of the following prostheses: an ankle prosthesis, an intervertebral disc prosthesis, a total wrist prosthesis and a partial wrist prosthesis.
